# EUROPÄISCHE PATENTANMELDUNG

(11) **EP 3 272 875 A1**
(43) Veröffentlichungstag der Anmeldung: **24.01.2018**
(21) Anmeldenummer: 17178717.9
(22) Anmeldetag: 29.06.2017
(51) Int. Cl.: C12Q 1/04, G01N 33/533

(54) **VERFAHREN ZUM NACHWEIS EINES BIOFILMS UND MITTEL ZUM NACHWEIS EINES BIOFILMS**

(30) Priorität: 18.07.2016 DE 102016113166
(71) Anmelder: Joanneum Research Forschungsgesellschaft mbH, 8010 Graz (AT)
(72) Erfinder: Mohr, Gerhard, 8010 Graz (AT)
(74) Vertreter: Strehl Schübel-Hopf & Partner

(57) **Zusammenfassung**

Die vorliegende Erfindung stellt ein allgemeines Verfahren zum Nachweisen von Biofilmen unter Einsatz von Indikatorfarbstoffen zur Bindung an Biofilmbestandteile bereit. Außerdem stellt die Erfindung ein Mittel, insbesondere ein Spray, Teststäbchen oder ein Verbandmaterial zum Nachweis von Biofilmen sowie ein das Verbandmaterial enthaltendes Pflaster bereit. Vorteil der vorliegenden Erfindung ist der selektive Nachweis und die einfache Durchführbarkeit des Verfahrens.

## Beschreibung

### Gebiet der Erfindung

Die vorliegende Erfindung betrifft ein allgemeines Verfahren zum Nachweisen von Biofilmen und ein Mittel zum Nachweis von Biofilmen, insbesondere ein Spray oder ein Verbandmaterial zum Nachweis von Biofilmen sowie ein das Verbandmaterial enthaltendes Pflaster.

### Stand der Technik

Biofilme bestehen aus einer Schleimschicht, auch Film genannt, in der Mikroorganismen eingebettet sind. Als Biofilm werden alle Aggregate von Mikroorganismen bezeichnet, die in eine von ihnen gebildete Schleimschicht eingebettet sind.

Biofilme bilden sich überwiegend in wässrigen Systemen, wenn sich dort Mikroorganismen an Grenzflächen ansiedeln. Grundsätzlich können alle Flächen von Biofilmen bewachsen werden.

An Grenzflächen bzw. Körperöffnungen von Tieren und Menschen gibt es häufig nichtkrankheitserregende Biofilm-Populationen. Beispiele hierfür sind die Bakteriengemeinschaften von Haut, Mund und Darm (Haut-, Mund- und Darmflora). Auch der Zahnbelag, der sich auf Zähnen bildet, stellt einen Biofilm dar. Die beteiligten Bakterien gehen mit dem Wirt eine interspezifische Wechselbeziehung ein, wobei die Bakterien eine Reihe von Aufgaben erfüllen. So sind sie bei der Reifung des Immunsystems in den ersten Lebensjahren von Bedeutung. Außerdem werden potenziell krankheitserregende Bakterien ferngehalten oder die Verdauungsprozesse unterstützt. Kommt es zu einem Ungleichgewicht in der Population, kann dies zu Krankheiten führen.

Ein Biofilm enthält außer den Mikroorganismen hauptsächlich Wasser. Von den Mikroorganismen ausgeschiedene extrazelluläre polymere Substanzen (EPS) bilden in Verbindung mit Wasser Hydrogele, so dass eine schleimartige Matrix entsteht, in der Nährstoffe und andere Substanzen gelöst sind. Oft werden von der Matrix auch anorganische Partikel oder Gasbläschen eingeschlossen. Die Gasphase kann je nach Art der Mikroorganismen mit Stickstoff, Kohlenstoffdioxid, Methan oder Schwefelwasserstoff angereichert sein.

Die EPS bestehen aus Biopolymeren, die in der Lage sind, Hydrogele zu bilden und die somit dem Biofilm eine stabile Form geben. Dabei handelt es sich um ein breites Spektrum von Polysacchariden, Proteinen, Lipiden und Nukleinsäuren.

Obwohl Biofilme in der Natur allgegenwärtig sind, wird ihre klinische Bedeutung in der Medizin häufig unterschätzt. Dies gilt insbesondere für Infektionen, denn in mehr als 60 % aller bakteriellen Infektionskrankheiten schützen sich die Erreger durch die Bildung von Biofilmen vor dem Immunsystem. Da ein großer Teil des anfänglichen mikrobiologischen Instrumentariums im Zuge großer Seuchen entwickelt wurde, geschah dies im Hinblick auf die frei schwebenden, sich schnell teilenden Bakterien akuter Infektionen. Die hier geforderte Isolation und Reinkultur im Labor führt allerdings zu beträchtlichem Genverlust der Bakterien unter herkömmlichen Laborbedingungen und schließlich zum Verlust der Fähigkeit zur Biofilmbildung. Deswegen entziehen sich Biofilme in der Akkumulationsphase häufig dem Nachweis durch konventionelle Verfahren der Mikroorganismenkultur. Moderne Techniken zur Visualisierung wie konfokale Mikroskopie und Gensonden zur Lokalisierung und Identifizierung von Biofilm-Organismen mittels Fluoreszenzmikroskopie haben zu einem besseren Verständnis der Biofilme beigetragen.

Im Zuge der Biofilmreifung kommt es in der Existenzphase, koordiniert durch das sogenannte Quorum sensing, zum Ablösen größerer Bakterienansammlungen. Dadurch entsteht eine Quelle für Keime, die zu chronischen und wiederkehrenden Infektionen von Patienten und unter Umständen bis hin zur häufig tödlichen Sepsis führen. Dies gilt insbesondere für Patienten mit geschwächtem Immunsystem. Dadurch kann es zu einer Reihe von Infektionen kommen.

Ein weiteres Feld sind die Fremdkörper-assoziierten Infektionen, z. B. durch die Besiedlung von Kathetern. Besonders wegen der Affinität verschiedener Mikroorganismen, wie einigen *Staphylokokken*, zu den Oberflächen von Biomaterialien sind etwa die Hälfte der nosokomialen Infektionen auf chirurgische Implantate zurückzuführen. Abhängig von dem verwendeten Medizinprodukt und der Verweildauer kommen gram-positive, gram-negative Bakterien und Pilze als Einzel- oder Multi-Spezies-Biofilm vor.

Aufgrund der teilweise ungeklärten erhöhten Antibiotikum-Resistenz der Bakterien im Biofilm ist in vielen Fällen die Entfernung des jeweiligen Implantats erforderlich.

Um die Kontamination von Wasser und Lebensmitteln, aber auch von Medikamenten und Kosmetika durch Mikroorganismen zu verhindern, sind ständige Maßnahmen gegen Biofilmbildung nötig. Dabei fallen jedes Jahr große Mengen durch Reinigungs- und Desinfektionsmittel belasteten Wassers an.

Bei der Wasseraufbereitung durch Membranverfahren sind Biofilme für das Biofouling verantwortlich, das bei dieser Technik zu schwerwiegenden Störungen führt. Ebenfalls unter Biofouling fallen Biofilme, die sich an Unterwasserkörpern bilden. Dies kann zu erheblichen Problemen führen. Beispielsweise können Biofilme durch einen erhöhten Reibungswiderstand die Geschwindigkeit eines Tankers erheblich verringern.

Aufgrund dieser Bedeutung von Biofilmen ist es notwendig, geeignete Mittel zum Nachweisen von Biofilmen zu entwickeln.

Ganz allgemein spielt die Fluoreszenzmarkierung von Biomolekülen eine wichtige Rolle in der Bioanalytik und biologischen Forschung.

Aus DE 10 2010 001 855 A1 sind Methoden zur Überwachung von Heilungsprozessen von Wunden sowie ein Verfahren zum Erkennen einer Änderung einer Eigenschaft einer Wunde bekannt. Das Verfahren verwendet einen Träger einer Substanz, die auf einer Änderung einer Eigenschaft einer Wunde mit einer Änderung seiner optischen oder chemischen Eigenschaften reagiert. Beispielsweise kann die Substanz bei einem pH-Wert von zwischen 6.5 und 7.5 eine erste Farbe haben und bei einem pH-Wert unter 6.5 oder über 7.5, der durch eine Absonderung der Wunde verursacht wird, eine zweite Farbe aufweisen.

Aus der DE 10 2011 003 517 A1 sind Indikatortextilien bekannt, mit denen die Wechselwirkung von Indikatorfarbstoffen mit Körperflüssigkeiten nachgewiesen werden kann. Es können Indikatorfarbstoffe eingesetzt werden, die Änderungen der optischen Eigenschaften bei Kontakt mit dem jeweiligen Analyten zeigen. Die Analyten können beispielsweise der pH-Wert, Saccharide oder Ionen sein. Durch diese Methode wird eine kovalente Färbung von Textilien mit Indikatorfarbstoffen erreicht.

Die WO 2012/072980 A1 offenbart Zusammensetzungen zum Nachweisen von Biofilmen. Darin wird die Anfärbung von Biofilmen auf lebensfähigem Gewebe, z. B. Haut, offenbart. Als Beispiele solcher Farbstoffe werden mehr oder weniger selektive organische Verbindungen genannt, wie Eosin, Erythrosin, Rose Bengal, Fluoresceinderivate, Rhodaminderivate, Phthalocyanine, Acridine, Oxazine, Chinolone, Adenosine, Triarylmethane, Phenothiaziniumderivate, Phenanthridinderivate, Pheophorbidderivate und Porphyrinderivate.

### Durch die Erfindung zu lösende Aufgaben

Die Aufgabe der vorliegenden Erfindung ist es, ein verbessertes und einfaches, vorzugsweise selektives Verfahren zum Nachweisen von Biofilmen bereitzustellen.

Die Aufgabe der vorliegenden Erfindung ist es außerdem, ein Mittel zum Nachweis von Biofilmen, insbesondere ein Spray oder ein Verbandmaterial oder ein Pflaster zum Nachweisen von Biofilmen bereitzustellen, das es erlaubt, den Heilungsprozess einer Wunde möglichst einfach zu überwachen.

### Zusammenfassende Darstellung der Erfindung

Diese Aufgabe wird durch das in den Ansprüchen definierte Verfahren und die in den Ansprüchen definierten Produkte gelöst.

Der Gegenstand der vorliegenden Erfindung ist insbesondere in den folgenden Punkten 1 bis 15 definiert:
1. Verfahren zum Nachweis von Biofilmen, wobei ein Biofilmbestandteil mit einem Indikator unter Änderung der optischen Eigenschaften des Indikators wechselwirkt.
2. Verfahren nach Punkt 1, wobei die Wechselwirkung eine kovalente Bindung oder eine Komplexierung ist.
3. Verfahren nach Punkt 1 oder 2, wobei die kovalente Bindung mit einer Amingruppe oder Ammoniumgruppe oder Thiolgruppe oder Aldehyd oder Ketongruppe, insbesondere eines Proteins oder Peptids, oder einer Hydroxygruppe, insbesondere eines Kohlenhydrats, des Biofilmbestandteils eingegangen wird, oder wobei der Indikator eine Calixaren-, Kryptand- oder Kronenetherfunktion aufweist und einen Komplex mit einer Ammoniumfunktion eines Proteins oder Peptids eingeht.
4. Verfahren nach einem der vorstehenden Punkte, wobei der Indikator ein Pyryliumsalz, eine Trifluoracetylgruppe, eine Tricyanovinylgruppe, eine Aldehydgruppe, eine Maleimidgruppe, Dicyanovinylgruppe, eine Hydrazingruppe, eine Amino- oder Diaminogruppe, eine Isothiocyanat- oder Isocyanatgruppe oder eine Boronsäure umfasst.
5. Verfahren nach einem der vorstehenden Punkte, wobei der Biofilmbestandteil ein Kohlenhydrat mit einer Diolgruppe ist und der Indikator eine Boronsäure ist, oder wobei der Biofilmbestandteil ein Protein oder Peptid ist und der Indikator ein Pyryliumsalz ist, das mit einer Amingruppe des Proteins oder Peptids eine kovalente Bindung eingeht.
6. Verfahren nach einem der vorstehenden Punkte, wobei die optische Eigenschaft die Absorptionswellenlänge, Absorptionsintensität, Emissionswellenlänge, Emissionsintensität oder Fluoreszenzabklingzeit ist.
7. Verfahren nach einem der vorstehenden Punkte, wobei die optische Eigenschaft eine mit dem menschlichen Auge erkennbare Färbung ist und/oder der Indikator ohne die Wechselwirkung mit dem Biofilmbestandteil farblos ist.
8. Verfahren nach einem der vorstehenden Punkte, wobei sich der Biofilm auf der Oberfläche eines menschlichen oder tierischen Körpers befindet.
9. Verfahren nach einem der vorstehenden Punkte, wobei der Indikator in einem Trägermaterial enthalten ist und er sich aus dem Trägermaterial zum Biofilm hinbewegt und/oder sich der Biofilmbestandteil aus dem Biofilm zu dem in dem Trägermaterial fixierten Indikator hinbewegt.
10. Verfahren nach einem der vorstehenden Punkte, wobei der Indikator in einem Spray enthalten ist.
11. Verfahren nach einem der vorstehenden Punkte, wobei der farblose Indikator, der nach Wechselwirkung mit dem Biofilm eine für das menschliche Auge erfassbare Färbung aufweist, in einem Pflaster enthalten ist.
12. Mittel, insbesondere ein Verbandmaterial, ein Spray, Teststäbchen oder Teststreifen, zum Nachweis eines Biofilms in oder an einer Wunde, das einen Indikator enthält, der mit einem Biofilmbestandteil unter Änderung der optischen Eigenschaften des Indikators wechselwirken kann.
13. Mittel nach Punkt 12, das ein Verbandmaterial ist, welches folgende Merkmale aufweist: ein Verbandgrundmaterial; und ein Kontrollelement, das in oder auf dem Verbandgrundmaterial angeordnet ist, so dass eine Kontrollfläche des Kontrollelements sichtbar ist und ein Bereich auf und neben der Wunde mit dem Kontrollelement in Kontakt ist, wenn das Verbandmaterial auf die Wunde aufgebracht ist, wobei das Kontrollelement einen Indikator enthält, der mit einem Biofilmbestandteil unter Änderung der optischen Eigenschaften des Indikators wechselwirken kann, wobei diese Änderung auf der Kontrollfläche sichtbar wird.
14. Ein Verbandmaterial gemäß Punkt 12 oder 13 umfassendes Pflaster.
15. Verfahren zum Nachweis eines Biofilms in oder an einer Wunde, das folgende Schritte umfasst:
   das Kontaktieren des Mittels nach Punkt 12 mit einer Wunde, insbesondere das Aufbringen eines Verbandmaterials nach Punkt 12 oder 13 oder eines Pflasters nach Punkt 14 auf eine Wunde; und das Überwachen des Mittels, insbesondere der Kontrollfläche des Verbandmaterials, um eine sichtbare Änderung und dadurch das Vorhandensein eines Biofilms zu erkennen.

### Vorteile der Erfindung

Durch die bevorzugte Verwendung von Indikatoren, die selektiv mit Biofilmbestandteilen wechselwirken, kann ein vorteilhaftes Verfahren bereitgestellt werden.

Das erfindungsgemäße Verfahren ist äußerst vielseitig anwendbar, da Biofilme an allen Flächen, Oberflächen und Grenzflächen vorkommen.

Ein Vorteil des erfindungsgemäßen Mittels zum Nachweisen von Biofilmen liegt in der einfachen Handhabung. Beispielsweise bringt die Verwendung eines Farbkontrollstreifens im Verbandmaterial Vorteile, insbesondere für die Patientenbetreuung durch das nichtmedizinische Personal, weil mögliche Erkrankungen frühzeitig erkannt werden können.

Für den Patienten ergeben sich also mehrere Vorteile. Man hat die Möglichkeit Komplikationen bei der Wundheilung ohne schmerzhaftes Entfernen der Verbände zu erkennen. Das führt zu einer Zeitersparnis bei der Wundenkontrolle, da statt der Entfernung des Verbandes und einer Probenentnahme die visuelle Überprüfung der Farbe des Kontrollstreifens genügt.

Mit der Farbänderung wird zudem eine leicht zu deutende Information erzeugt, die es sogar dem Laien ermöglicht festzustellen, dass in der Wunde eine Veränderung stattgefunden hat. Dieser Umstand trägt zur Früherkennung von Komplikationen bei.

### Ausführungsformen der Erfindung

Die vorliegende Erfindung betrifft nach Punkt 1 ein Verfahren zum Nachweis von Biofilmen, wobei ein Biofilmbestandteil mit einem Indikator unter Änderung der optischen Eigenschaften des Indikators wechselwirkt.

Der hier verwendete Ausdruck "Biofilm" bedeutet eine Schleimschicht oder Film, worin Mikroorganismen oder Aggregate von Mikroorganismen eingebettet sind.

Der hier verwendete Ausdruck "Biofilmbestandteil" bedeutet jede Substanz und Verbindung, die in einem Biofilm, also der Schleimschicht, enthalten sein oder darin entstehen kann, wie verschiedene Makromoleküle, z. B. Cytoplasma eines Bakteriums mit Cytoplasmamembran, die interzellulare Glykokalyx mit Exo-Polysacchariden, DNA, hydrophobe und wasserlösliche Proteine, Periplasmamembran, Zellwand, Periplasma, Cytoplasmamembran und Cytoplasma eines Bakteriums, oder deren Abbauprodukte, wie Peptide, Aminosäuren, Oligosaccharide oder Monosaccharide. Außerdem umfasst der Ausdruck auch gasförmige Stoffe. Insbesondere können als Biofilmbestandteile Amine, Kohlendioxid, Alkohole, Aldehyde, Carbonsäuren, reaktive Sauerstoffspezies (inklusive Wasserstoffperoxid), Saccharide, Thiole, Diole, Stickoxide, Ketone, phosphororganische Verbindungen, Kationen, Anionen, Kohlenmonoxid und Aminosäuren genannt werden.

Der Biofilmbestandteil wechselwirkt direkt mit dem Indikator. Das heißt, dass der Biofilmbestandteil eine Bindung mit dem Indikator eingeht. Diese Bindung kann auf einer kovalenten Bindung, einen Komplexierung, einen hydrophoben Wechselwirkung, einer ionischen Wechselwirkung, auf Wasserstoffbrückenbindungen und dergleichen beruhen. Bevorzugt ist eine kovalente Bindung oder eine Komplexierung. Die Bindung ist so stark, dass sie in dem jeweils vorliegenden Medium zu der gewünschten Änderung der optischen Eigenschaften des Indikators führt.

Das erfindungsgemäße Verfahren ist äußerst vielseitig anwendbar, da Biofilme überall vorkommen. Beispielsweise ist das Verfahren anwendbar für Böden und Sedimente, auf Gestein, auf und in Pflanzen, Tieren und Menschen, hier insbesondere an den Schleimhäuten und chronischen Wunden. Das Verfahren ist anwendbar auf wässrige Systeme, wenn sich dort Mikroorganismen an Grenzflächen ansiedeln, und auf alle Flächen, die von Biofilmen bewachsen werden, z. B. Grenzflächen zwischen Gas- und Flüssigphasen (z. B. freier Wasserspiegel), Flüssig- und Festphasen (z. B. Kies an der Gewässersohle) oder auch zwischen verschiedenen Flüssigphasen (z. B. Öltröpfchen im Wasser). Eine besondere Anwendung findet das Verfahren an Oberflächen, Grenzflächen oder Körperöffnungen von Menschen und Tieren. Beispiele hierfür sind Haut, Mund, Schleimhäute und Darm, sowie Plaque, also Zahnbelag.

Da Biofilme mit einer Reihe von Infektionen in Verbindung gebracht werden, z. B. Osteomyelitis, Wundinfektionen, bakterielle Endokarditis, Parodontitis, Urethritis, Prostatitis, Zahnkaries, Periimplantitis, chronische Mittelohrentzündung bei Kindern, kann das erfindungsgemäße Nachweisverfahren im Zusammenhang mit diesen Infektionen eingesetzt werden.

Ein weiteres Anwendungsfeld umfasst Fremdkörper-assoziierte Infektionen. Hierunter fallen die mikrobielle Kontamination und Besiedlung von Kathetern, Implantaten und medizinischen Instrumenten, insbesondere aus Kunststoffen.

Als Ausgangspunkt der beteiligten Mikroorganismen gelten die Hautoberfläche von Krankenhauspersonal und Patienten, der Kontakt von Austrittsstellen oder Konnektoren mit Leitungswasser und weitere Quellen aus der Umgebung. Auch die Wasserleitungen und Dialyse-Ausrüstung sowie Endoskope können betroffen sein.

Beispiele für häufig von Fremdkörper-assoziierten Infektionen betroffene Medizinprodukte sind Venenkatheter, künstliche Herzklappen, Stoma, Gelenkprothesen, Peritonealdialyse-Katheter, Herzschrittmacher, Endotrachealtubi, Stimmprothesen, Zerebrospinalflüssigkeit-Shunts, Zahnimplantate.

Ebenfalls betroffen sind Sensorsysteme für Forschungs- oder Überwachungszwecke im maritimen Bereich, bei denen ein Bewuchs zu Funktionsbeeinträchtigungen führen kann.

In Verdunstungskühlanlagen können Biofilme zur Gesundheitsbeeinträchtigung der Beschäftigten führen. Insbesondere bei Reinigungs- und Instandhaltungsarbeiten kann es zu direktem Kontakt mit Haut und Atemwegen kommen.

Das erfindungsgemäße Verfahren kann in allen diesen Bereichen eingesetzt werden, um auf einfache Weise das Vorhandensein von Biofilmen nachzuweisen oder auszuschließen.

In einer Ausführungsform in dem erfindungsgemäßen Verfahren ist die optische Eigenschaft eine mit dem menschlichen Auge erkennbare Färbung und/oder ist der Indikator ohne die Wechselwirkung mit dem Biofilmbestandteil farblos.

Das heißt, der Indikator ist farblos und wird durch die Wechselwirkung mit dem Biofilmbestandteil gefärbt; oder der Indikator ist gefärbt und ändert seine Farbe durch die Wechselwirkung mit dem Biofilmbestandteil; oder der Indikator ist gefärbt und wird durch die Wechselwirkung mit dem Biofilmbestandteil entfärbt.

Der hier verwendete Ausdruck "mit dem menschlichen Auge erkennbare Färbung" oder für das menschliche Auge "sichtbar" oder allgemein "Färbung" bedeutet, dass die Färbung in einem Wellenlängenbereich liegen kann, der von dem menschlichen Auge ohne Hilfsmittel wahrgenommen werden kann. Dieser Wellenlängenbereich ist von 350 bis 750 nm, insbesondere von 400 bis 700 nm.

In einer Ausführungsform befindet sich in dem erfindungsgemäßen Verfahren der Biofilm auf der Oberfläche eines menschlichen oder tierischen Körpers. Das bedeutet, dass der Biofilm auf der Oberfläche des menschlichen Körpers entsteht, insbesondere auf der Haut, auf den Schleimhäuten oder in, an oder auf Wunden.

In einer Ausführungsform ist in dem erfindungsgemäßen Verfahren oder Mittel der Indikator in einem Trägermaterial enthalten und bewegt sich aus dem Trägermaterial zum Biofilm hin und/oder der Biofilmbestandteil bewegt sich aus dem Biofilm zu dem in dem Trägermaterial fixierten Indikator hin. Das heißt in einer ersten Alternative, dass der Indikator in dem Trägermaterial enthalten ist, aber nicht kovalent oder in sonstiger Weise fixiert ist. Vielmehr ist er beispielsweise in einer Flüssigkeit enthalten und kann mit dieser Flüssigkeit aus dem Trägermaterial abgesondert werden oder aus der Flüssigkeit diffundieren. Diese Alternative wird vorzugsweise dann angewandt, wenn der zu erfassende Biofilmbestandteil hochmolekular ist und daher eher fest und unbeweglich in der Biofilmmatrix gebunden ist. In einer zweiten Alternative ist der Indikator auf dem oder in dem Trägermaterial fest fixiert, so dass es vorzugsweise auch zu keinem Ausbluten oder Austrag des Indikators kommt. Diese Fixierung kann durch kovalente Bindung erfolgen, durch Fixierung an Partikel, wie weiter unter beschrieben, oder durch Einschluss in ein Kompartiment, dessen Außenwand nicht für den Indikator, jedoch für den zu erfassenden Biofilmbestandteil, durchlässig ist. Eine dritte Alternative ist eine Kombination der ersten beiden Alternativen dahingehend, dass sowohl der Indikator als auch der Biofilmbestandteil frei beweglich sind.

In einer Ausführungsform ist in dem erfindungsgemäßen Verfahren oder Mittel der Indikator in einer Flüssigkeit enthalten. Vorzugsweise ist der Indikator in einem Spray enthalten. Beispielsweise dient als Flüssigkeit, insbesondere als Lösungsmittel, ein Alkohol wie Isopropanol. Dieser kann zudem gleichzeitig als Desinfektionsmittel dienen, wenn die Anwendung im medizinischen Bereich erfolgt.

Außerdem kann der Indikator in einer Zusammensetzung enthalten sein, die zwei Funktionen hat. Die eine Funktion ist der Nachweis von Biofilmen, die andere Funktion ist die Entfernung, Auflösung oder Zerstörung von Biofilmen. Auf diese Weise wird gleichzeitig mit der Durchführung der Maßnahme zur Entfernung von Biofilmen der Erfolg dieser Maßnahme überprüft. In einer Ausführungsform sind diese beiden Funktionen in einer Verbindung oder einem Komplex oder Verbund vereint.

In einer Ausführungsform ist in dem erfindungsgemäßen Verfahren oder Mittel der farblose Indikator, der nach Wechselwirkung mit dem Biofilmbestandteil eine für das menschliche Auge erfassbare Färbung aufweist, in einem Pflaster enthalten. Das Pflaster bedeckt vorzugsweise eine Wunde. Vorzugsweise ist der Indikator so in dem Pflaster enthalten, dass ein Farbumschlag erfasst werden kann oder sichtbar wird, ohne dass das Pflaster entfernt werden muss.

In der vorliegenden Erfindung umfasst der Ausdruck "Wunde" eine Verletzung zumindest der Hautoberfläche eines Patienten oder eine sonstige infektionsanfällige Störung auf der Oberfläche eines Patienten.

Der in der vorliegenden Erfindung verwendete Ausdruck "Patient" meint einen Menschen oder ein Tier von beliebigem Gesundheitszustand, d. h. auch gesunde, insbesondere auch von einem Biofilm nicht betroffene Menschen oder Tiere, die beispielsweise nur vorsorglich auf das Vorhandensein eines Biofilms untersucht werden.

### Indikatorfarbstoffe

Die Indikatorfarbstoffe (die im Folgenden manchmal einfach nur als Indikatoren bezeichnet werden) tragen spezifische Rezeptorgruppen, um eine selektive Biofilmbestandteilerkennung zu gewährleisten.

Ein Beispiel zur Erfassung von Aminen ist die Trifluoracetylgruppe-Amin-Wechselwirkung. Rezeptoren, die eine sichtbare Änderung der Kontrollfläche für reaktive Sauerstoffspezies hervorrufen, können zum Beispiel Metall-Ligand-Komplexe verwendet werden und die Erkennungsreaktion dann am Metallzentrum stattfindet. Fluorescein-Sulfonatgruppen sind selektive Rezeptorgruppen für Wasserstoffperoxid, Maleimidfunktionen sind selektive Rezeptorgruppen für Thiole, Tricyanovinylgruppen sind selektive Rezeptorgruppen für primäre und sekundäre Amine, Pyrylium-Funktionen sind selektive Rezeptorgruppen für primäre Amine, Hydrazinfunktionen sind selektive Rezeptorgruppen für Aldehyde und Ketone, Aminogruppen sind selektive Rezeptorgruppen für Aldehyde und Ketone, Boronsäurefunktionen sind selektive Rezeptorgruppen für Diole, 1,2-Diaminobenzolgruppen sind selektive Rezeptorgruppen für Stickoxide, Aldehydfunktionen sind selektive Rezeptorgruppen für Aminosäuren, Isocyanate sind selektive Rezeptorgruppen für Alkohole, Isothiocyanate sind selektive Rezeptorgruppen für Amine und Alkohole. Kronenether, Calixarene und Kryptanden sind selektive Rezeptorgruppen für Kationen und Anionen, Metallpolyphyrinkomplexe sind selektive Rezeptorfunktionen für Kohlenmonoxid, Amine und Stickoxide.

Beispiele von in der vorliegenden Erfindung einsetzbaren Indikatoren sind Fluorophore, welche die genannten Rezeptoren oder Rezeptorengruppen tragen. Fluorophore können mit Biomolekülen, z.B. Biofilmbestandteilen, unter Änderung der optischen Eigenschaften wechselwirken. Man unterscheidet zwischen Fluorophoren, die nicht-kovalent an Biomoleküle wie z. B. Proteine oder DNA binden, und solchen, die kovalent an Biomoleküle gebunden werden können. Es gibt Marker, die kovalent an Biomoleküle mit darin vorhandenen Aminogruppen binden können.

Das allgemeine Reaktionsschema bei allen bekannten Verfahren kann wie folgt dargestellt werden: An einem Fluorophor F befindet sich eine Gruppe X (Rezeptorgruppe), die mit einer an einem Biomolekül befindlichen zweiten Gruppe (z. B. HY) eine chemische Reaktion (oder auch nur eine starke Bindung wie zum Beispiel zwischen Biotin und Avidin) eingeht. Bei der Bildung chemischer (kovalenter) Bindungen wird dabei eine Gruppe vom Typ XH abgespalten, typischerweise nach folgender Reaktionsgleichung:

F-X + HY-Biomolekül -> F-Y-Biomolekül + XH.

Die Konjugation kann aber auch im Sinn einer Additionsreaktion unter Bildung der Verbindung F-XH-Y-Biomolekül erfolgen. Typische Beispiele der Gruppen X und Y und der Reaktionstypen sind folgende:

| **X (am Fluorophor)** | **Y (am Biomolekül)** | **Reaktionstyp (Abgangsgruppe bzw. neue Gruppe)** |
|---|---|---|
| -SO₂Cl | R'-NH₂ | Substitution (-HCl) |
| -CO-CH₂-l | R'-SH | Substitution (-HI) |
| -CO-CH₂-Br | R'-COOH | Substitution (-HBr) |
| -CO-O-NHS*) | R'-NH₂ | Substitution (-N-Hydroxysuccinimid) |
| -NCS | R'-NH₂ | Addition (-NH-CS-NH-R') |
| -NCO | R'-OH (Alkohol) | Addition (-NH-CO-OR') |
| -Maleinimid | R'-SH | Addition |
| -Biotin | R'-Avidin | Affinitätsbindung (nicht kovalent) |
| -Avidin | R'-Biotin | Affinitätsbindung (nicht kovalent) |
| -Pyrylium | R'-NH₂ | Substitution (-H₂O) |
| -Boronsäure | R'-OH (Saccharid) | Substitution (-H₂O) |
| -Kronenether | R'-NH₃⁺ | Komplexbindung |
| -Calixaren | R'-NH₃⁺ | Komplexbindung |
| -Kryptand | R'-NH₃⁺ | Komplexbindung |
| -COCF₃ | R'-NH₂ | Addition |
| -CHO | R'-NH₂ | Addition |
| -Tricyanovinyl | R'-NH₂ | Substitution |
| -Dicyanovinyl | R'SH | Addition |
| -NH₂ | Aldehyd/Keton | Addition |
| -NH-NH₂ | Aldehyd/Keton | Addition |
| -(NH₂)₂ | Stickoxide | Addition |

| | | |
|---|---|---|
| *)NHS = N-Hydroxysuccinimid | | |

Auf diese Weise gelingt es, einen Fluorophor F in ein Molekül einzuführen und dieses somit allen fluoreszenzanalytischen Verfahren zugänglich zu machen.

Der vorstehend genannte Fluorophor F kann durch einen beliebigen anderen Indikator ersetzt werden. Das heißt, die Rezeptorgruppe X ist nicht auf die Bindung an einen Fluorophor beschränkt. Vielmehr kann sie an einen beliebigen, in der vorliegenden Erfindung eingesetzten Indikator gebunden sein, der bei Wechselwirkung mit dem Biofilmbestandteil seine optischen Eigenschaften ändert.

Wie vorstehend beschrieben wurde, gibt es verschiedene Arten, ein Biomolekül mit einem Indikator, z.B. einem Fluoreszenzmarker, zu versehen.

Bekannte geeignete Bausteine zur Kopplung mit Aminen, die gerade in der medizinischen Chemie eine Rolle spielen, sind Pyrylium-Salze, da sich ein primäres Amin in den Aromaten unter Bildung eines Pyridinium-Salzes einbauen kann. Als Amin kann eine Aminosäure, ein organisches Amin, eine Protein oder ein aminomodifiziertes Nucleotid oder dessen Oligomer genannt werden.

Die Pyryliumgruppe kann mit einem oder mehreren Resten R substituiert sein, wobei die Reste R an der Pyryliumgruppe vorzugsweise Wasserstoff oder Kohlenwasserstoffreste mit 1 bis 30 C-Atomen, vorzugsweise 1 bis 10 C-Atomen sind. Beispiele für besonders bevorzugte Reste R sind Wasserstoff, Methyl, tert-Butyl und Phenyl. Besonders bevorzugt sind Pyryliumverbindungen, in denen ein Fluorophor F in Paraposition angeordnet ist. Weiterhin ist bevorzugt, dass der Rest R in den Position 2 und 6 (ortho) von Wasserstoff verschieden ist.

Beispiele von Pyryliumsalzen und deren Umsetzungsprodukten mit Aminverbindungen sowie die Änderungen der Fluoreszenzeigenschaften sind in EP 1 308 728 A2 offenbart.

Andere bekannte Indikatorgruppen zur Kopplung mit organischen Verbindungen sind Boronsäuren R-B(OH)2. Boronsäuren sind Derivate der Borsäure, bei denen eine Hydroxygruppe durch eine Organylgruppe R substituiert ist. Die Organylgruppe kann mindestens eine Gruppe enthalten, die unter einer Acetonylgruppe, Acylgruppe (z. B. Acetylgruppe, Benzoylgruppe), Alkylgruppe (z. B. Methylgruppe, Ethylgruppe), Alkenylgruppe (z. B. Vinylgruppe, Allylgruppe), Alkinylgruppe (Propargylgruppe), Aminocarbonylgruppe, Arylgruppe (z. B. Phenylgruppe, 1-Naphthylgruppe, 2-Naphthylgruppe, 2-Thiophenylgruppe, 2,4-Dinitrophenylgruppe), Alkylarylgruppe (z. B. Benzylgruppe, Triphenylmethylgruppe), Benzyloxycarbonylgruppe (Cbz), *tert-*Butoxycarbonylgruppe (Boc), Carboxygruppe, (Fluoren-9-ylmethoxy)carbonylgruppe (Fmoc), Furfurylgruppe, Glycidylgruppe, Halogenalkylgruppe (z. B. Chlormethylgruppe, 2,2,2-Trifluorethylgruppe), Indolylgruppe, Nitrilgruppe, Nucleosidylgruppe und Tritylgruppe ausgewählt ist.

Sie zählen damit zu den bororganischen Verbindungen. Ein einzigartiges Merkmal der Boronsäuren ist, dass sie zusammen mit Sacchariden, Aminosäuren und Hydroxamsäuren reversible kovalente Komplexe bilden können. Typische Anwendungen für Boronsäuren sind die Anbindung an Saccharide zur Fluoreszenzdetektion.

Beispiele für Indikatoren für den kolorimetrischen Nachweis von Kohlenhydraten sind ortho-Azo-substituierte Phenylboronsäuren (Egawa, Y. et al. (2007) "Ortho-azo substituted phenylboronic acids for colorimetric sugar sensors", Bioorganic & Medicinal Chemistry Letters 20 17, 3789-3792). Die kovalente Bindung des Indikators an Diole, z. B. Glukose, führt zu einer Änderung des UV-Spektrums im sichtbaren Bereich, insbesondere bei 398 nm und 521 nm.

Weitere Indikatoren auf Boronsäurebasis sind offenbart in Trupp, S. et al. (2006) "Fluororeactands for the Detection of Saccharides Based on Hemicyanine Dyes with a Boronic Acid Receptor", Microchim Acta 153, 127-131. Die darin genannten Hemicyaninfarbstoffe binden kovalent an Zucker, z. B. Fruktose, was zu einer Änderung der Absorption und der Fluoreszenz im sichtbaren Wellenlängenbereich führt.

Weitere Indikatoren auf Boronsäurebasis sind offenbart in Trupp, S. et al. (2006) "A fluorescent water-soluble naphthalimide-based receptor for saccharides with highest sensitivity in the physiological pH range", Org. Biomol. Chem. 4, 2965-2968. Der darin genannte Rezeptor bindet kovalent an Zucker, was zu einer Änderung der Absorption und der Fluoreszenz im sichtbaren Wellenlängenbereich führt.

Weitere Indikatoren auf Boronsäurebasis sind offenbart in Ward, C. J. (2002) "Boronic acid appended azo dyes-colour sensors for saccharides", J. Chem. Soc., Perkin Trans. 1, 462-470. Die darin genannten Boronsäureazofarbstoffe komplexieren in wässriger Lösung mit Monosacchariden, was zu einer Änderung der Absorption im sichtbaren Wellenlängenbereich führt.

Die vorstehend im Stand der Technik genannten Boronsäureverbindungen können in dem erfindungsgemäßen Verfahren eingesetzt werden.

Weitere Ausführungsbeispiele der vorliegenden Erfindung beziehen sich auf einen Indikatorstreifen (bzw. Sensorfolie). Im Falle von Wundabdeckungen kann der Indikatorstreifen (Sensorfolie) in die Innenseite der Abdeckung integriert werden und beispielsweise auf biogene Amine reagieren. Gelangen diese nun in die Luft zwischen Wunde und Abdeckung, so reagiert der Indikatorfarbstoff des Indikatorstreifens mit ihnen und wechselt seine Farbe, z. B. von gelb zu blau. Die Farbänderung des Indikatorstreifens kann somit eine Warnfunktion übernehmen.

Da in einigen erfindungsgemäßen Ausführungsformen der Indikator in den Biofilm eindringen muss, um mit dem Biofilmbestandteil wechselwirken zu können, kann es erforderlich sein, den Indikator zu funktionalisieren, z. B. hydrophob oder hydrophil zu machen, indem beispielsweise langen Alkylreste angehängt werden, oder mit positiven oder negativen Ladungen zu versehen.

### Mittel zum Nachweis eines Biofilms

Die vorliegende Erfindung betrifft auch ein Mittel zum Nachweis eines Biofilms, insbesondere eines Biofilms auf, an oder in einer Wunde. Dieses Mittel enthält einen in der vorliegenden Erfindung genannten Indikator, der unter Änderung seiner optischen Eigenschaften mit einem Biofilmbestandteil wechselwirkt. Dieses Mittel ist beispielsweise ein Verbandmaterial, ein Spray, ein Teststäbchen oder ein Teststreifen. Das Teststäbchen kann ein Wattestäbchen sein. Dieses Mittel ist in einer Ausgestaltung so ausgeführt, dass es für die Anwendung im Bereich einer Wunde geeignet ist. Insbesondere ist das Mittel, wenn es in direkten Kontakt mit der Wunde kommt, ungiftig und hautverträglich, also medizinisch geeignet. In einem Spray kann als Lösungsmittel ein Alkohol wie Isopropanol eingesetzt werden. Dieser kann zudem gleichzeitig als Desinfektionsmittel dienen. Die Teststäbchen oder Teststreifen können mit einem der genannten Indikatoren zum Nachweisen von Biofilmen in gelöster Form getränkt sein.

In dem erfindungsgemäßen Mittel kann der Indikator kovalent oder nicht kovalent an ein geeignetes Material des Mittels angebunden werden. Die Bindung erfolgt beispielsweise über im Mittel vorhandene Nano- und/oder Mikropartikel, die unten näher beschrieben sind.

Außerdem kann der Indikator an die weiter unten beispielhaft für das Verbandmaterial beschriebenen Fasern gebunden sein.

Im Folgenden wird das erfindungsgemäße Mittel anhand eines erfindungsgemäßen Verbandmaterials eingehend beschrieben.

Die für das Verbandmaterial beschriebenen Ausgestaltungen und Vorteile gelten gleichermaßen entsprechend für die anderen erfindungsgemäßen Mittel, insbesondere die Teststeifen und Teststäbchen wie Wattestäbchen.

### Verbandmaterial

Eine Ausführungsform der vorliegenden Erfindung ist ein Verbandmaterial zum Nachweis eines Biofilms in, auf oder an einer Wunde, das einen Indikator enthält, der mit einem Biofilmbestandteil unter Änderung der optischen Eigenschaften des Indikators wechselwirken kann.

Das Verbandmaterial kann in einem Pflaster enthalten sein.

Der hier verwendete Ausdruck "Verbandmaterial" ist jede Art von Material zur Abdeckung oder zum Schützen einer Wunde. Das Verbandmaterial deckt vorzugsweise einen Bereich ab, der die Wunde sowie einen Bereich um die Wunde umfasst.

Das Verbandmaterial umfasst vorzugsweise ein Verbandgrundmaterial und ein Kontrollelement. Das Kontrollelement enthält den in der vorliegenden Erfindung einsetzbaren Indikator, der mit einem Biofilmbestandteil unter Änderung der optischen Eigenschaften des Indikators wechselwirkt. Das Kontrollelement ist in oder auf dem Verbandgrundmaterial angeordnet, so dass eine Kontrollfläche des Kontrollelements sichtbar ist und eine flüssige oder gasförmige Absonderung einer Wunde mit dem Kontrollelement in Kontakt ist, wenn das Verbandmaterial auf die Wunde aufgebracht ist. Des Weiteren ist das Kontrollelement so ausgelegt, um bei einer Entstehung eines Biofilms in oder an der Wunde, basierend auf einer chemischen Reaktion, eine sichtbare Änderung der Kontrollfläche hervorzurufen, um die Überwachung der Wundheilung zu ermöglichen.

Ausführungsbeispiele gemäß der Erfindung basieren auf dem Kerngedanken, ein Kontrollelement, das auf die Entstehung eines Biofilms mit einer sichtbaren Änderung einer Kontrollfläche reagiert, so anzuordnen, dass diese Entstehung nachgewiesen werden kann, ohne das Verbandmaterial von der Wunde zu entfernen. Dadurch können Zeit und Geld gespart werden. Zusätzlich wird der Heilungsprozess nicht durch das Entfernen gestört.

Da die Änderung bereits von außen sichtbar ist, kann eine Störung des Heilungsprozesses auf einfache Art und Weise und mit geringem Aufwand erkannt werden.

Ausführungsbeispiele gemäß der Erfindung werden nachfolgend näher erläutert.

Das Verbandmaterial umfasst ein Verbandgrundmaterial und ein Kontrollelement. Das Kontrollelement ist in oder auf dem Verbandgrundmaterial angeordnet, so dass eine Kontrollfläche des Kontrollelements sichtbar ist und eine flüssige oder gasförmige Absonderung einer Wunde mit dem Kontrollelement in Kontakt ist, wenn das Verbandmaterial auf die Wunde aufgebracht ist. Zusätzlich ruft das Kontrollelement bei einer Änderung einer Eigenschaft der Wunde, basierend auf einer chemischen Reaktion eine sichtbare Änderung der Kontrollfläche hervor, um die Überwachung der Wundheilung zu ermöglichen.

Durch die beschriebene Anordnung des Kontrollelements kann gewährleistet werden, dass eine Änderung der Kontrollfläche, verursacht durch die Entstehung eines Biofilms, sichtbar ist, obwohl sich das Verbandmaterial noch auf der Wunde befindet. In anderen Worten, das Kontrollelement ist in oder auf dem Verbandgrundmaterial so angeordnet, dass die Wundheilung überwachbar ist, ohne das Verbandmaterial von der Wunde zu entfernen.

Dadurch kann auf eine einfache Art und Weise und mit geringem Aufwand die Wundheilung verfolgt werden. Durch das beschriebene Konzept kann die Wundheilung beispielsweise ohne Verbandswechsel und ohne Verwendung diagnostischer Blattmaterialien überwacht werden.

Zusätzlich kann das Kontrollelement ausgelegt sein, eine Stärke der sichtbaren Änderung der Kontrollfläche proportional zu einer Stärke der Entstehung eines Biofilms hervorzurufen.

Beispielsweise kann sich die Kontrollfläche umso stärker verfärben, je größer oder dichter der Biofilm ist. Dadurch kann ein Anhaltspunkt für das Ausmaß einer Störung einer Wundheilung ermöglicht werden.

Das Verbandmaterial kann beispielsweise ein Pflaster oder ein Verband sein. Bei dem Verbandgrundmaterial kann es sich um jegliches bekanntes Material, das beispielsweise zur Herstellung von Pflaster oder von Verbänden verwendet wird, handeln. Dabei kann das Verbandgrundmaterial aus einem oder mehreren verschiedenen Materialien bestehen.

Das Kontrollelement ist beispielsweise ein Träger einer Substanz, die auf einen Biofilmbestandteil, basierend auf einer chemischen Reaktion z. B. mit einer Änderung seiner optischen oder chemischen Eigenschaften reagiert. Beispielsweise kann die Substanz, verursacht durch die Entstehung eines Biofilms, ihre Farbe, ihre Helligkeit, ihre Fluoreszenz, ihre Lumineszenz oder ihre chemische Reaktivität mit anderen Stoffen ändern. Dies kann direkte sichtbare Änderungen der Kontrollfläche hervorrufen, wenn die Substanz beispielsweise direkt auf die Kontrollfläche aufgebracht ist, oder indirekt eine sichtbare Änderung der Kontrollfläche bewirken.

Die hier genannte Substanz kann ein in der vorliegenden Erfindung beschriebener Indikator sein.

Die Kontrollfläche des Kontrollelements kann auf einer der Wunde abgewandten Seite des Kontrollelements oder auf einer der Wunde zugewandten Seite des Kontrollelements angeordnet sein. Ist die Kontrollfläche auf der der Wunde zugewandten Seite des Kontrollelements angeordnet, so kann das Kontrollelement zumindest teilweise durchsichtig oder zumindest zu einem ausreichenden Grade transparent sein, so dass die Kontrollfläche sichtbar ist, wenn das Verbandmaterial auf die Wunde aufgebracht ist. Zum Beispiel kann das Kontrollelement ein biokompatibles Polymer oder ein Zellstoff sein. Der Indikator kann dann beispielsweise auf der Kontrollfläche des Kontrollelements gebunden sein. Zusätzlich kann das biokompatible Polymer durchsichtig sein, wenn die Kontrollfläche auf der der Wunde zugewandten Seite des Polymers angeordnet ist.

Das Verbandgrundmaterial kann eine beliebige Form, wie beispielsweise Formen von herkömmlichen Pflastern oder Verbanden oder andere Formen, aufweisen. Das Kontrollelement ist in oder auf dem Verbandgrundmaterial angeordnet. Dabei ist zumindest eine Kontrollfläche des Kontrollelements sichtbar.

Das Kontrollelement kann auf dem Verbandgrundmaterial oder in dem Verbandgrundmaterial angeordnet sein, so dass zumindest ein Teil des Verbandgrundmaterials (oder das Verbandgrundmaterial mit einem Teil seiner Dicke) zwischen dem Kontrollelement und der Wunde angeordnet ist, wenn das Verbandmaterial auf die Wunde aufgebracht ist.

Das Verbandmaterial kann eine Zwischenschicht umfassen. Die Zwischenschicht ist zwischen dem Kontrollelement und der Wunde angeordnet, wenn das Verbandmaterial auf die Wunde aufgebracht ist. Die Zwischenschicht kann beispielsweise ein Material aufweisen, das einen guten Transport von flüssigen oder gasförmigen Absonderungen von dem Biofilm zu dem Kontrollelement ermöglicht. Dies kann beispielsweise aufgrund einer Kapillarwirkung eines Zellstoffs passieren.

Das Verbandmaterial kann zusätzlich eine Trägerschicht umfassen. Die Trägerschicht ist auf der auf der Wunde abgewandten Seite des Verbandgrundmaterials und des Kontrollelements angeordnet, und dient beispielsweise zur Fixierung der Position des Kontrollelements in oder auf dem Verbandgrundmaterial. Die Trägerschicht ist zumindest in einem Bereich des Kontrollelements durchsichtig oder zumindest zu einem Grad transparent, so dass die Kontrollfläche des Kontrollelements sichtbar ist.

Das Verbandmaterial kann auch ein großflächiges Kontrollelement umfassen, durch das eine Überwachung von größeren Wunden ermöglicht wird. Es kann zusätzlich zur Beurteilung, ob eine Änderung einer Eigenschaft der Wunde stattgefunden hat, auch eine Aussage über den Ort der Änderung und/oder das Ausmaß der Änderung ermöglichen.

Tritt nur in einem Teil der Wunde ein Biofilm auf, ist neben der Information, ob ein Biofilm auftritt, auch eine Aussage über den Ort der Entstehung und/oder das Ausmaß der Entstehung wichtig.

Die sichtbare Änderung der Kontrollfläche kann sich auf einen Teil der Kontrollfläche beschränken. Der Ort der sichtbaren Änderung kann mit dem Ort der Wunde korreliert werden, in dem sich die Eigenschaft der Wunde ändert, und die Größe der sichtbaren Änderung kann proportional zu der Fläche der Wunde sein, in der sich die Eigenschaft der Wunde ändert. Dadurch kann eine Aussage über den Ort und/oder das räumliche Ausmaß einer Biofilmentstehung gemacht werden.

Die Verwendung eines biokompatiblen Polymers und die kovalente Immobilisierung des Indikators bei der Herstellung des Kontrollstreifens kann die Kontamination der Wunde mit schädlichen Substanzen verhindern.

Ein Vorteil eines Verbandmaterials mit Farbkontrollstreifen liegt in der einfachen Handhabung. Eine Verwendung eines solchen Kontrollstreifens im Verbandmaterial könnte große Vorteile bei der Patientenbetreuung mit sich bringen. Das beschriebene Konzept kann die Patientenbetreuung für das nichtmedizinische Personal vereinfachen, weil mögliche Erkrankungen frühzeitig erkannt werden können.

Das erfindungsgemäße Verbandmaterial kann beispielsweise auch für Patienten mit großflächigen Wunden (z. B. auch Brandopfer) eine Verbesserung mit sich bringen, da gerade dort die Wundkontrolle besonders zeitaufwendig und schmerzhaft ist und ein häufiges Öffnen des Wundverbandes ein großes Infektionsrisiko birgt.

Wird der Kontrollstreifen in Pflastern für den häuslichen Gebrauch eingesetzt, kann er z. B. die Wundkontrolle bei Kindern vereinfachen. Eltern haben so die Möglichkeit, ihren Kindern die Entfernung des Pflasters zu ersparen, da auch hier z. B. die visuelle Überprüfung der Farbe des Kontrollstreifens genügt.

Für den Patienten ergeben sich also mehrere Vorteile. Man hat die Möglichkeit Komplikationen bei der Wundheilung ohne schmerzhaftes Entfernen der Verbände zu erkennen. Das führt zu einer Zeitersparnis bei der Wundkontrolle, da statt der Entfernung des Verbandes und einer Probenentnahme die visuelle Überprüfung der Farbe des Kontrollstreifens genügt.

Mit der Farbänderung wird zudem eine leicht zu deutende Information erzeugt, die es sogar dem Laien (und dem Patienten) ermöglicht, festzustellen, dass in der Wunde eine Veränderung stattgefunden hat. Dieser Umstand trägt zur Früherkennung von Komplikationen bei.

Durch das beschriebene Konzept kann beispielsweise ein intelligentes Pflaster zur optischen Wundüberwachung bereitgestellt werden. Angestrebt ist eine Vereinfachung der Wundenkontrolle durch Einbringen eines Kontrollstreifens (Kontrollelement) in das Verbandmaterial. Der Kontrollstreifen soll z. B. durch einen einfachen Farbumschlag Veränderungen in der Wunde anzeigen. Eine Farbänderung eines Kontrollstreifens im Verband kann auch von nichtmedizinischem Personal und auch vom Patienten leicht erkannt werden.

Die chemische Anbindung der reaktiven Indikatorfarbstoffe kann z. B. durch chemische Reaktion der OH-Gruppen der Zellulosefasern des Verbandmaterials mit Vinylsulfonylgruppen der Indikatorfarbstoffe erfolgen, wie es in DE 10 2011 003 517 A1 beschrieben ist.

### Bindung der Indikatoren an Fasern des Verbandmaterials

Gemäß einer Ausführungsform ist der Indikator an das Verbandmaterial gebunden.

Eine stabile chemische Bindung von Indikatorfarbstoffen an Fasern kann z. B. durch die Verwendung aminofunktionalisierter Fasern und die chemische Bindung einer Säurefunktion des Indikatorfarbstoffes an die Aminogruppen der Fasern des Verbandmaterials erzielt werden.

Die dadurch geknüpfte Amidbindung ist sehr stabil und sorgt ebenfalls für das dauerhafte Färben der Fasern mit Indikatorfarbstoffen.

Ein Vorteil obiger Verbandmaterialien, deren Fasern mit chemisch gebundenen Indikatorfarbstoffen ausgestattet sind, ist die einfache Handhabung und der hohe Informationsgehalt der beispielsweise Farbänderung.

Bezüglich des Ortes der Anwendung der Verbandmaterialien mit Fasern, die mit chemisch gebundenen Indikatorfarbstoffen ausgestattet sind, besteht ein wichtiger Vorteil in der chemischen Bindung und damit sehr hohen Stabilität der Färbung. Die Materialien können direkt auf der Hautoberfläche der Personen getragen werden, da aufgrund der stabilen Haftung der Fasern ein Austragen der Farbstoffe nicht erfolgt.

Ein weiterer Vorteil ist die Möglichkeit, zur Früherkennung von Biofilmen beizutragen, indem entsprechende Indikatoren an die Fasern gebunden werden.

Die vorstehend beschriebene Bindung der Indikatoren an Fasern ist jedoch nicht auf das Verbandmaterial beschränkt, sondern umfasst alle erfindungsgemäßen Mittel zum Nachweis von Biofilmen. Wenn dieses Mittel beispielsweise ein Teststäbchen ist, das mit der Wunde in Berührung kommt, können diese Teststäbchen Fasern enthalten, an denen der Indikator gebunden ist.

### Bindung der Indikatoren an Partikel

Gemäß einer alternativen Ausführungsform können die Indikatorfarbstoffe nicht unmittelbar, sondern mittelbar per chemischer Bindung an die Fasern angebunden sein, wie z. B. indem dieselben kovalent an Partikel, gebunden werden/sind, wobei diese Partikel ihrerseits wiederum kovalent oder nichtkovalent an die Fasern des Verbandmaterials gebunden, gedruckt, geklebt oder laminiert werden/sind bzw. anders mit dem Verbandmaterial verbunden sind.

Das Material der Partikel kann aus anorganischen Komponenten, beispielsweise aus Kieselsäuren, Titandioxid, Siliciumdioxid, Zirkoniumphosphat, Zeolithen, Silika, Gold, Silber oder Glas, oder aus organischen Materialien, vorzugsweise Polymeren bestehen, wie Cellulose und Cellulosederivaten wie Carboxymethylcellulose, Hydroxypropylcellulose, Hydroxypropylmethylcellulose, Dextran, Polyacrylamid, gelierte Stärke, Gelatine, Polylactat, Polystyrol oder Polyvinylpyrrolidon, oder die vorgenannten Materialien können als Komponente in diesen Partikeln enthalten sein, oder Hybridmaterialien sein, wie zum Beispiel organischmodifizierte Sol-Gel Gläser.

Die in der vorliegenden Erfindung eingesetzten Partikel sind Nano- und/oder Mikropartikel und weisen beispielsweise einen mittleren Durchmesser auf, der zwischen 10 Nanometer und 100 Mikrometer, jeweils einschließlich, liegt. Die Partikel können sphärisch geformt sein. Um die Farbstoffe chemisch stabil an Partikel zu binden, können sie beispielsweise kovalent an die Oberfläche oder in Poren der Partikel gebunden werden. Alternativ ist es möglich, dass die Partikel im Rahmen ihrer Herstellung in die Partikel kovalent einpolymerisiert werden. Dadurch können die Partikel in einem separaten Herstellungsprozess mit dem Farbstoff belegt werden, und danach in den üblichen Druckverfahren auf die Textilien aufgedruckt werden. Diese Textilien können beispielsweise Wundverbände sein.

In einer weiteren Ausführungsform können die Partikel Bestandteil eines Sprays sein, das in die Wunde gesprüht werden kann. Hierbei wählt der Fachmann selbstverständlich solche Materialen für die Partikel, die biokompatibel sind, wie TiO₂, Polylactat, Kieselsäure oder Stärke.

Eine Ausführungsform der vorliegenden Erfindung ist ein Wundpflaster mit einem Indikatorbereich, der Partikel aufweist, an die ein Indikatorfarbstoff zum Detektieren eines Biofilmbestandteils unmittelbar gebunden ist. Die nachstehenden Ausführungen, die auf ein Wundpflaster bezogen sind, gelten gleichermaßen für jegliche Art von Wundabdeckung, z. B. einen Verband.

Bei Ausführungsbeispielen kann der Indikatorfarbstoff durch die unmittelbare Bindung an die Partikeln einfach gehandhabt und vielseitig verwendet werden. Beispielsweise ist eine einfache Aufbringung des Indikatorfarbstoffes mit den Partikeln an oder in das Wundpflaster möglich. Bei der Herstellung kann der Indikatorfarbstoff mit Partikeln z. B. als Suspension vorliegen und auf das Wundpflaster gesprüht, gedruckt oder gerakelt werden. Ferner kann die Suspension als Folie gezogen und die Folie auf das Wundpflaster aufgebracht werden. Des Weiteren führt die unmittelbare Bindung des Indikatorfarbstoffs an die Partikel zu einem gegenüber bekannten Systemen schnelleren Ansprechverhalten des Indikatorfarbstoffs durch kürzere Diffusionswege.

Es kann durch die unmittelbare Bindung des Indikatorfarbstoffs an die Partikel ein Wundpflaster mit einem einfach handhabbaren und vielseitig verwendbaren Indikatorbereich geschaffen werden. Beispielsweise wird es ermöglicht, ohne Abziehen des Wundpflasters oder Öffnen des Verbandes einen Biofilm nachzuweisen.

Hierzu kann ein wie vorstehend beschriebener Indikatorfarbstoff eingesetzt werden.

Der Biofilmbestandteil kann beispielsweise ein biogenes Amin sein, welches bei einem Zersetzungsprozess entsteht, wobei der Indikatorfarbstoff mit dem Biofilmbestandteil reagiert und seine Farbe wechselt.

Der Indikatorfarbstoff kann mittels einer chemischen Bindung unmittelbar an Partikel gebunden sein. Dabei kann die chemische Bindung z. B. eine Atombindung bzw. kovalente Bindung sein. Im Falle einer Atombindung ist der Indikatorfarbstoff fest an die Partikel gebunden. Dadurch kann ein Austreten des Indikatorfarbstoffs aus dem Indikatorbereich, der die Partikel aufweist, und eine damit verbundene eventuelle Kontamination der Wunde vermieden werden. Ferner kann die Wundabdeckung in dem Indikatorbereich eine semipermeable Sperrschicht aufweisen, die z. B. ausgebildet sein kann, um die Biofilmbestandteile durchzulassen. Durch die semipermeable Sperrschicht kann darüber hinaus ein Austreten von Partikeln aus dem Indikatorbereich vermieden werden.

Bei Ausführungsbeispielen können die Partikel, an die der Indikatorfarbstoff zum Detektieren des Biofilmbestandteils unmittelbar gebunden sind, zu einem Indikatorstreifen verarbeitet werden, der auf die Wundabdeckung aufgebracht oder in die Wundabdeckung integriert werden kann. In diesem Fall weist der Indikatorbereich der Wundabdeckung den Indikatorstreifen auf.

Eine Freisetzung des Biofilmbestandteils kann dabei optisch mit dem Indikatorstreifen (basierend beispielsweise auf Absorption, Reflexion, Lumineszenz, SPR (SPR = Surface Plasmon Resonance, dt. Oberflächen-Plasmon-Resonanz) oder IR (IR = infrarot Absorption oder Raman Absorption)), der z. B. in die Wundabdeckung integriert ist, nachgewiesen werden. Dies kann erreicht werden durch die Herstellung von in die Wundabdeckung integrierbaren Indikatorstreifen, die auf verschiedene Arten von Biofilmbestandteilen anschlagen. Hierfür können geeignete reversible oder irreversibel arbeitende Indikatorfarbstoffe mit entsprechenden Rezeptorfunktionalitäten verwendet werden. Diese maßgeschneiderten Indikatorfarbstoffe werden an die Partikel aus geeigneten organischen oder anorganischen Polymeren chemisch stabil (kovalent) gebunden. Wie bereits erwähnt, können die Partikel anschließend entweder direkt, oder in eine geeignete natürliche oder künstliche Polymermatrix eingebettet, zu einem Indikatorstreifen verarbeitet werden. Der Indikatorstreifen kann bei Bedarf durch eine geeignete semipermeable Sperrschicht ergänzt werden. Die stabile chemische Immobilisierung der Indikatorfarbstoffe kann eine mögliche Kontamination der Wunde verhindern, des Weiteren kann die Sperrschicht eine mögliche Migration von Polymer- und Farbstoffbestandteilen in die Wunde unterdrücken.

Der wichtigste Vorteil der Wundabdeckung mit dem Indikatorstreifen basierend auf Partikel ist die einfache Handhabung und vielseitige Verwendbarkeit. Die Verwendung eines solchen Indikatorstreifens ermöglicht es dem Patienten oder dem Klinikpersonal, schnell und einfach ohne Öffnen der Wundabdeckung eine Aussage über eine Infektion der Wunde zu treffen.

Die Verwendung von Partikeln hat gegenüber bekannten Systemen den Vorteil von rascherem Ansprechverhalten durch kürzere Diffusionswege. Durch die kleinen Dimensionen der Partikel, die z. B. im Bereich von 10 bis 150 nm oder im Bereich von 3 bis 600 nm liegen können, ergibt sich eine schnellere Reaktion des eingebetteten Indikatorfarbstoffs auf die Biofilmbestandteile verglichen mit der Einbettung dickerer Polymermatrizes.

Ferner können die Partikel porös sein. Es können somit Selektivitäten gegenüber den Biofilmbestandteilen, und somit die Verlässlichkeit der Farbänderung durch die Porosität der Partikel beeinflusst werden. Hier kann je nach Porosität oder Art der Poren die Diffusion des gewünschten Biofilmbestandteils vorteilhaft gesteuert werden. Dadurch ist eine starke Verbesserung der Selektivität des Indikatorstreifens auf wenige Biofilmbestandteile möglich.

So ist bei Partikeln auch eine Verwendung von mehreren Farbstoffen gleichzeitig, eingebettet in verschiedene Partikel, möglich. Diese können mehrere Biofilmbestandteile parallel erkennen. Alternativ kann bei Lumineszenzmessungen ein inerter Farbstoff zur Referenzierung des Signals zugesetzt werden. Dies erhöht die Verlässlichkeit der Aussage des Indikatorstreifens weiter.

Ferner können die Partikel einen Kern und eine Hülle aufweisen. In anderen Worten, die Partikel können in einem Kern-Hülle-Aufbau gestaltet werden. Dabei kann der Kern des Partikels den Indikatorfarbstoff aufweisen, und die Hülle des Partikels eine Löslichkeit des Partikels in Polymermaterialien steuern.

Alternativ kann die Hülle des Partikels Poren aufweisen, wobei die Poren für ein Gas selektiv durchlässig sind, und wobei der Kern des Partikels den Indikatorfarbstoff aufweist, wobei der Indikatorfarbstoff ausgebildet ist, um das Gas in seiner ionischen Form zu detektieren. Somit kann die Hülle eine spezielle Porosität aufweisen, welche nur Gase selektiv durchlässt, während sich in dem Kern ein Indikatorfarbstoff für Ionen befindet, der das Gas dann in seiner ionischen Form detektiert.

Alternativ kann die Hülle des Partikels ein Enzym zur chemischen Umsetzung eines Biofilmbestandteils aufweisen, wobei bei der chemischen Umsetzung ein Produkt entsteht, und wobei der Kern des Partikels den Indikatorfarbstoff und einen Referenzfarbstoff aufweist, wobei der Indikatorfarbstoff ausgebildet ist, um das Produkt zu detektieren. Somit kann im Kern ein Referenzfarbstoff und ein Indikatorfarbstoff vorliegen, während sich in der Hülle ein Enzym befindet, welches den zu untersuchenden Biofilmbestandteil chemisch umsetzt, weil diese Reaktion in der Folge ein Produkt erzeugt, welches dann vom Indikator detektiert wird.

Vor allem bietet die Verwendung der Partikel die Möglichkeit einer stabilen Immobilisierung des Indikatorfarbstoffs und dadurch ein Unterdrücken der Kontamination der Wunde. Der Indikatorfarbstoff kann chemisch stabil (kovalent) in die Matrix des partikelformenden Polymers eingearbeitet werden. Somit können diese nicht mehr aus den Partikeln austreten und die Wunde kontaminieren. Dies trägt stark zur Sicherheit des Patienten bei. Die Partikel können wahlweise in eine Polymerschicht eingebracht, quervernetzt oder direkt auf ein Trägermaterial, wie zum Beispiel eine Folie, aufgebracht werden.

Bei der Herstellung der Wundabdeckung ist es zum Beispiel möglich, die Partikel mit (geeigneten) Polymeren zu einer Folie, einem Netz oder Schaum zu extrudieren, und die Folie, das Netz oder den Schaum auf die Wundabdeckung in dem Indikatorbereich aufzubringen. Darüber hinaus ist es bei der Herstellung der Wundabdeckung möglich, die Partikel mit (geeigneten) Polymeren zu der Wundabdeckung zu extrudieren.

Ein großer Vorteil von Partikeln ist somit ihre deutlich verbesserte Prozessierbarkeit gegenüber anderen Formulierungen, was sich in ihrer guten Verarbeitbarkeit in Rolle-zu-Rolle-Verfahren, Ink-Jet-Drucken, Siebdrucken und Extrudieren zeigt. Dies ist ein großer Vorteil auf eine günstige großtechnische Fertigung des Indikatorstreifens.

Die vorstehend beschriebene Bindung der Indikatoren an Partikel ist jedoch nicht auf das Verbandmaterial beschränkt, sondern umfasst alle erfindungsgemäßen Mittel zum Nachweis von Biofilmen. Wenn dieses Mittel beispielsweise ein Teststäbchen ist, das mit der Wunde in Berührung kommt, können diese Teststäbchen Partikel enthalten, an denen der Indikator gebunden ist. Ein anderes Beispiel ist ein Spray, worin der Indikator an die beschriebenen Partikel gebunden ist.

## Patentansprüche

1. Verfahren zum Nachweis von Biofilmen, wobei ein Biofilmbestandteil mit einem Indikator unter Änderung der optischen Eigenschaften des Indikators wechselwirkt.

2. Verfahren nach Anspruch 1, wobei die Wechselwirkung eine kovalente Bindung oder eine Komplexierung ist.

3. Verfahren nach Anspruch 1 oder 2, wobei die kovalente Bindung mit einer Amingruppe oder Ammoniumgruppe oder Thiolgruppe oder Aldehyd oder Ketongruppe, insbesondere eines Proteins oder Peptids, oder einer Hydroxygruppe, insbesondere eines Kohlenhydrats, des Biofilmbestandteils eingegangen wird, oder wobei der Indikator eine Calixaren-, Kryptand- oder Kronenetherfunktion aufweist und einen Komplex mit einer Ammoniumfunktion eines Proteins oder Peptids eingeht.

4. Verfahren nach einem der vorstehenden Ansprüche, wobei der Indikator ein Pyryliumsalz, eine Trifluoracetylgruppe, eine Tricyanovinylgruppe, eine Aldehydgruppe, eine Maleimidgruppe, Dicyanovinylgruppe, eine Hydrazingruppe, eine Amino- oder Diaminogruppe, eine Isothiocyanat- oder Isocyanatgruppe, oder eine Boronsäure umfasst.

5. Verfahren nach einem der vorstehenden Ansprüche, wobei der Biofilmbestandteil ein Kohlenhydrat mit einer Diolgruppe ist und der Indikator eine Boronsäure ist, oder wobei der Biofilmbestandteil ein Protein oder Peptid ist und der Indikator ein Pyryliumsalz ist, das mit einer Amingruppe des Proteins oder Peptids eine kovalente Bindung eingeht.

6. Verfahren nach einem der vorstehenden Ansprüche, wobei die optische Eigenschaft die Absorptionswellenlänge, Absorptionsintensität, Emissionswellenlänge, Emissionsintensität oder Fluoreszenzabklingzeit ist.

7. Verfahren nach einem der vorstehenden Ansprüche, wobei die optische Eigenschaft eine mit dem menschlichen Auge erkennbare Färbung ist und/oder der Indikator ohne die Wechselwirkung mit dem Biofilmbestandteil farblos ist.

8. Verfahren nach einem der vorstehenden Ansprüche, wobei sich der Biofilm auf der Oberfläche eines menschlichen oder tierischen Körpers oder auf einem Implantat oder medizinischen Gerät befindet.

9. Verfahren nach einem der vorstehenden Ansprüche, wobei der Indikator in einem Trägermaterial enthalten ist und er sich aus dem Trägermaterial zum Biofilm hinbewegt und/oder sich der Biofilmbestandteil aus dem Biofilm zu dem in dem Trägermaterial fixierten Indikator hinbewegt.

10. Verfahren nach einem der vorstehenden Ansprüche, wobei der Indikator in einem Spray enthalten ist.

11. Verfahren nach einem der vorstehenden Ansprüche, wobei der farblose Indikator, der nach Wechselwirkung mit dem Biofilm eine für das menschliche Auge erfassbare Färbung aufweist, in einem Pflaster enthalten ist.

12. Mittel, insbesondere ein Verbandmaterial, ein Spray, Teststäbchen oder ein Teststreifen, zum Nachweis eines Biofilms in, auf oder an einer Wunde, das einen Indikator enthält, der mit einem Biofilmbestandteil unter Änderung der optischen Eigenschaften des Indikators wechselwirken kann.

13. Mittel nach Anspruch 12, das ein Verbandmaterial ist, welches folgende Merkmale aufweist:
ein Verbandgrundmaterial; und
ein Kontrollelement, das in oder auf dem Verbandgrundmaterial angeordnet ist, so dass eine Kontrollfläche des Kontrollelements sichtbar ist und ein Bereich auf und neben der Wunde mit dem Kontrollelement in Kontakt ist, wenn das Verbandmaterial auf die Wunde aufgebracht ist,
wobei das Kontrollelement einen Indikator enthält, der mit einem Biofilmbestandteil unter Änderung der optischen Eigenschaften des Indikators wechselwirken kann, wobei diese Änderung auf der Kontrollfläche sichtbar wird.

14. Mittel nach Anspruch 12, das ein Spray ist, wobei das Spray ein Lösungs- oder Suspensionsmittel, vorzugsweise Alkohol, Wasser oder eine Alkohol-Wasser-Mischung, sowie den in dem Lösungsmittel gelösten Indikator oder den an Partikeln kovalent oder nicht-kovalent angebundenen Indikator umfasst, wobei die Partikel im Suspensionsmittel suspendiert vorliegen.

15. Ein Verbandmaterial gemäß Anspruch 12 oder 13 umfassendes Pflaster.

16. Verfahren zum Nachweis eines Biofilms in oder an einer Wunde, das folgende Schritte umfasst:
das Kontaktieren des Mittels nach Anspruch 12 mit einer Wunde, insbesondere das Aufbringen eines Verbandmaterials nach Anspruch 12 oder 13 oder eines Pflasters nach Anspruch 14 auf eine Wunde, oder das Besprühen der Wunde mit einem Spray nach Anspruch 12 oder 14; und
das Überwachen des Mittels, insbesondere der Kontrollfläche des Verbandmaterials, um eine sichtbare Änderung und dadurch das Vorhandensein eines Biofilms zu erkennen.
